Europäisches Patentamt

European Patent Office

Office européen des brevets

(11) Publication number: **0 201 153**
A2

(12) # EUROPEAN PATENT APPLICATION

(21) Application number: 86300788.6

(22) Date of filing: **06.02.86**

(51) Int. Cl.⁴: **C 12 N 15/00**, C 12 N 9/50,
A 61 K 37/54
// C12N9/64, C12N9/70

(30) Priority: **09.02.85 GB 8503358**
**16.01.86 GB 8600979**

(43) Date of publication of application: **12.11.86**
**Bulletin 86/46**

(84) Designated Contracting States: **BE CH DE FR GB IT LI LU NL SE**

(71) Applicant: **BEECHAM GROUP PLC, Beecham House Great West Road, Brentford Middlesex TW8 9BD (GB)**

(72) Inventor: **Robinson, Jeffery Hugh, 3 Hillcrest Close Epsom, Surrey KT18 5JY (GB)**
Inventor: **Browne, Michael Joseph, 7 Delaporte Close Epsom, Surrey KT17 1AF (GB)**
Inventor: **Tyrrell, Arthur William Russell, White Friars 38 St. Albans Road, Reigate Surrrey RH2 9LN (GB)**

(74) Representative: **Valentine, Jill Barbara et al, Beecham Pharmaceuticals Patents & Trade Marks Dept. Great Burgh Yew Tree Bottom Road, Epsom Surrey KT18 5XQ (GB)**

(54) **Modified enzyme and process for its preparation.**

(57) A modified tissue-type plasminogen activator comprising an amino acid without a charged side chain in place of lysine at position 277.

EP 0 201 153 A2

## Novel Compounds

The present invention relates to a modified
fibrinolytic enzyme in particular modified tissue-type
plasminogen activator, preparation of the modified
enzyme, pharmaceutical compositions containing the
modified enzyme and its use in the treatment of
thrombotic disease.

Tissue-type plasminogen activator (t-PA) is known to
have useful fibrinolytic activity.  The sequence of
amino acids which make up the enzyme and the nucleotide
sequence of the cDNA which codes for t-PA are known
(see e.g. Pennica et al, Nature, 1983, 301, 214-221).

However one disadvantage of the use of this enzyme in
the treatment of thrombotic disease is that it can
activate circulating plasminogen in addition to
fibrin-bound plasminogen.  This may cause unwanted
side-effects such as indiscriminate haemorrhaging.

The applicants have now identified a modified t-PA
enzyme which retains fibrinolytic activity but in which
the activity is more specific.

According to the present invention there is provided
a modified tissue-type plasminogen activator comprising
an amino acid without a charged side chain in place of
lysine at position 277.

In a preferred aspect, the amino acid without a charged
side chain at position 277 is isoleucine.

- 2 -

As used herein, the term tissue-type plasminogen activator denotes a plasminogen activator of the group having the immunological properties defined for t-PA at the XXVIII Meeting of the International Committee on Thrombosis and Haemostasis, Bergamo, Italy, 27 July 1982.

The amino acid sequence of various forms of t-PA are known.  The abovementioned Nature 1983 reference discloses the sequence for the L-chain and the mature S-chain forms of t-PA, also discussed by Vehar et.al., Biotechnology, 1984, 2, 1051-7 in which the processing of initially formed t-PA by removal of a pro-sequence to give the S-chain form is reported.  Pohl et.al., FEBS letters, 1984, Vol. 168 No.1, 29-32, refers to the N-terminal multiplicity of t-PA and discloses the U-chain form.  The numbering system for the amino acid sequence of t-PA used herein is that described in the Nature 1983 reference for mature (S-chain) t-PA in which the N-terminal serine is numbered 1.  By this system, L-chain t-PA has an N-terminal glycine residue at position -3 and U-chain t-PA has an N-terminal valine at position 4.  It is understood that the tissue-type plasminogen activator modified in accordance with the present invention encompasses all such variant forms.

The modified t-PA of the invention may be derivatised to provide pharmaceutically useful conjugates analogous to known t-PA-containing conjugates, for example:

(a)  an enzyme-protein conjugate as disclosed in EP-A-O 155 388, in which the catalytic site on the enzyme which is responsible for fibrinolytic activity is blocked by a human protein attached thereto by way of a reversible linking group;

- 3 -

(b) an enzyme-protein conjugate as disclosed in EP-A-O 152,736, comprising at least one optionally blocked fibrinolytic enzyme linked by way of a site other than the catalytic site responsible for fibrinolytic activity to at least one human protein;

(c) a protein-polymer conjugate as disclosed in co-pending application no. 85308533.0 comprising a pharmaceutically useful protein linked to at least one water soluble polymer by means of a reversible linking group; or

(d) an enzyme conjugate as disclosed in co-pending application no. 85308534.8 comprising a plurality of fibrinolytic enzymes linked together through the active centres thereof by means of a removable blocking group.

The modified t-PA of the invention may take the place of t-PA as the (pro) enzyme or (human) protein component, as appropriate, of any of the conjugates described above.

The modified t-PA of the invention or conjugate thereof can be further derivatised such that any catalytic site essential for fibrinolytic activity is optionally blocked by a removable blocking group.

The above mentioned derivatives of the modified t-PA may be used in any of the methods and compositions described hereinafter for the modified t-PA itself.

As used herein the expression 'removable blocking group' includes groups which are removable by hydrolysis at a rate such that the pseudo-first order

- 4 -

rate constant for hydrolysis is in the range of $10^{-6}$ sec$^{-1}$ to $10^{-3}$ sec$^{-1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$ alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$ alkylphenyl.

In a further aspect, the invention provides a process for preparing modified tissue-type plasminogen activator according to the invention which process comprises expressing DNA encoding said modified tissue-type plasminogen activator in a recombinant host cell and recovering the modified tissue-type plasminogen activator product.

The DNA polymer comprising a nucleotide sequence that encodes the modified t-PA also forms part of the invention.

The process of the invention may be performed by conventional recombinant techniques such as described in Maniatis et. al., Molecular Cloning - A Laboratory Manual; Cold Spring Harbor, 1982.

In particular, the process may comprise the steps of:

- 5 -

i.) preparing a replicable expression vector
capable, in a host cell, of expressing a DNA
polymer comprising a nucleotide sequence that
encodes said modified tissue-type plasminogen
activator;

ii) transforming a host cell with said vector;

iii) culturing said transformed host cell under
conditions permitting expression of said DNA
polymer to produce said modified tissue-type
plasminogen activator; and

iv) recovering said modified tissue-type
plasminogen activator.


The invention also provides a process for preparing the
DNA polymer by the condensation of appropriate mono-,
di- or oligomeric nucleotide units.

The preparation may be carried out chemically,
enzymatically, or by a combination of the two methods,
in vitro or in vivo as appropriate. Thus, the DNA
polymer may be prepared by the enzymatic ligation of
chemically synthesised DNA fragments, by conventional
methods such as those described by D. M. Roberts et al
in Biochemistry 1985, 24, 5090-5098. The chemical
synthesis may be performed generally as described
hereinafter for the synthesis of an
oligodeoxyribonucleotide. Enzymatic polymerisation of
DNA may be carried out in vitro using a DNA polymerase
such as DNA polymerase I (Klenow fragment) in an
appropriate buffer containing the nucleoside
triphosphates dATP, dCTP, dGTP and dTTP as required at

- 6 -

a temperature of $10^{\circ}-37^{\circ}C$, generally in a volume of $50\mu l$ or less. Enzymatic ligation of DNA fragments may be carried out using a DNA ligase such as T4 DNA ligase in an appropriate buffer at a temperature of $4^{\circ}C$ to ambient, generally in a volume of $50\mu l$ or less. DNA polymer which encodes the modified t-PA may be prepared by site-directed mutagenesis of the cDNA which codes for tissue-type plasminogen activator, by conventional methods such as those described by G. Winter et al in Nature 1982, 299, 756-758 or by Zoller and Smith 1982; Nucl. Acids Res., 10, 6487-6500.

The site-directed mutagenesis may be effected by means of an oligodeoxyribonucleotide which is complementary or corresponds to tissue-type plasminogen activator cDNA in the region on either side of the codon formed by the nucleotides 1018-1020 but which mismatches in the region 1018-1020 such that the triplet of the oligodeoxyribonucleotide in the mismatch region is complementary or corresponds to a codon which encodes an amino acid without a charged side chain.

The oligodeoxyribonucleotide also forms part of the invention.

According to the present invention there is further provided a process for preparing a modified tissue-type plasminogen activator enzyme, which process comprises effecting a site-directed mutagenesis upon the cDNA which codes for tissue-type plasminogen activator using an oligonucleotide which is complementary or corresponds to the t-PA cDNA in the region on either side of the codon formed by the nucleotides 1018-1020 but which mismatches in the region 1018-1020 such that cDNA is produced which contains a codon suitable for producing an amino acid without a charged side chain at this position; and expressing the modified cDNA in a prokaryote or eukaryote host.

Further according to the present invention there is provided modified tissue-type plasminogen activator obtained by the above described process.

In a preferred aspect, the triplet in the mismatch region of the oligodeoxyribonucleotide is complementary or corresponds to a codon which encodes isoleucine.

The invention also provides a process for preparing an oligodeoxyribonucleotide of the invention, which process comprises the deprotection of a second oligodeoxyribonucleotide having the same sequence of bases as said oligodeoxyribonucleotide and in which all free hydroxy and amino groups are protected.

The oligodeoxyribonucleotides used in the process can be prepared by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H.

- 8 -

Koester, Nucleic Acids Research, 1984, 12, 4539; and
H.W.D. Matthes et al., EMBO Journal, 1984, 3, 801.

Solid supports which may be employed include
conventional supports known in the art, for example
kieselguhr-polydimethylacrylamide, silica,
controlled-pore glass, or cellulose paper discs. The
base at the 3′-end of the oligodeoxyribonucleotide to
be prepared is attached, via a spacer group connected
to the 3′-oxygen of the terminal 2′-deoxyribose unit,
to the solid support and assembly of the
oligodeoxyribo-nucleotide (in a protected form) is
carried out in the 3′-> 5′-direction by cycles of
synthesis, the required operations being performed
either manually or by an automated instrument.

At the end of the synthesis, the protected
oligodeoxyribonucleotide may be cleaved from the solid
support either before, after, or at the same time as
other deprotection steps that are carried out once the
desired sequence of bases has been assembled, as
described hereinbelow. Conveniently the
oligodeoxyribonucleotide is removed from the solid
support by treatment with a basic reagent such as
aqueous ammonia at ambient or slightly elevated
temperature, or with 1,1,3,3 -tetramethyl-
guanidinium-syn- 2-nitrobenzaldoximate or similar
reagents in aqueous dioxan at ambient or slightly
elevated temperature.

Alternatively, the preparation may be carried out
conventionally by phosphotriester chemistry in solution
as described, for example, by C.B. Reese, ·Tetrahedron,
1978, 34, 3143-3179.

The required sequence of nucleotide bases in the oligodeoxyribonucleotide may be built up conventionally, for
example as described in the aforementioned
publications, by the condensation of appropriate mono-,
di- or oligomeric nucleotide units in the presence of a
suitable condensing agent such as 1-(mesitylene-
sulphonyl)-3- nitro-1,2,4-triazole in a solvent such as
pyridine at ambient or slightly elevated temperature
(when phosphotriester chemistry is employed) or by a
condensing agent such as tetrazole in acetonitrile at
ambient temperature (when phosphite or phosphoramidite
chemistry is used). Optionally a 'capping' step is
introduced after each condensation step to inactivate
any unreacted starting material containing a free
5′-hydroxy group. Such a capping step may suitably be
carried out by an acylating agent such as acetic
anhydride in 2,6-lutidine and
4-$\underline{N}$,$\underline{N}$-dimethylamino-pyridine in tetrahydrofuran at
ambient temperature.

When phosphite or phosphoramidite chemistry is
employed, the phosphorus (III) atom in the
phosphite-triester internucleotide linkage created in
each condensation step is oxidised, giving the
corresponding phosphotriester linkage, before a further
condensation step is carried out. Such oxidations may
be carried out using a convenient oxidising agent, for
example iodine in aqueous tetrahydrofuran in the
presence of a base such as lutidine. The oxidation
step is conveniently carried out after the optional
'capping' step as hereinbefore described.

During the synthesis of the oligodeoxyribonucleotide,
the hydroxy groups in each of the internucleotide
phosphodiester bridges may be protected by an aryl group or alkyl group conventionally employed for the purpose, for example 2- or 4-chlorophenyl, methyl, or 2-cyanoethyl, to prevent branching of the molecule. The aryl protecting groups are removed at the end of the synthesis by treatment with an agent conventionally employed for the purpose, for example 1,1,3,3-tetramethyl-guanidinium- _syn_ -2-nitrobenzaldoximate in an aqueous solvent such as aqueous dioxan at ambient temperature. The methyl groups may be removed at the end of the synthesis by treatment with triethylammonium thiophenolate in dioxan at ambient temperature. The 2-cyanoethyl groups may be removed at the end of the synthesis with a basic reagent such as triethylamine or tert-butylamine in pyridine at ambient temperature, or alternatively by treatment with concentrated aqueous ammonia at about $50^{o}C$ (a step which simultaneously and advantageously removes the protecting groups present on the A, C and G bases as described hereinbelow).

The amino groups present in the A, C and G bases are protected by base-labile protecting groups, such as benzoyl for A and C, and isobutyryl for G. Such groups may be removed by treatment with basic reagents, for example ammonia at ambient or slightly elevated temperature, for example $50^{o}C$. The 5'-hydroxy group of the terminal ribose moiety is protected by an acid-labile group such as trityl, 4,4'-dimethoxytrityl or pixyl (9-phenyl-9-xanthyl), which is removed before each condensation step and at the end of synthesis. Such groups may be removed by treatment with an acidic reagent such as di- or trichloroacetic acid in an anhydrous solvent such as dichloromethane or chloroform, at ambient temperature.

- 11 -

The expression of the DNA polymer encoding the modified t-PA in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and combining said linear segment with the DNA polymer encoding the modified t-PA under ligating conditions.

The choice of vector will be determined in part by the host cell, which may be prokaryotic or eukaryotic. Suitable vectors include plasmids and bacteriophages.

The preparation of the replicable expression vector may be carried out conventionally with appropriate enzymes for restriction, polymerisation and ligation of the DNA, by procedures described in, for example, Maniatis et al cited above. Polymerisation and ligation may be performed as described above for the preparation of the DNA polymer. Digestion with restriction enzymes may be performed in an appropriate buffer at a temperature of $20^{\circ}-70^{\circ}C$, generally in a volume of 50µl or less with 0.1-10µg DNA.

The recombinant host cell is prepared, in accordance with the invention, by transforming a host cell with a replicable expression vector of the invention under transforming conditions. Suitable transforming conditions are conventional and are described in, for example, Maniatis et al cited above, or ''DNA Cloning'' Vol. II, D.M. Glover ed., IRL Press Ltd, 1985.

- 12 -

The choice of transforming conditions is determined by the host cell. Thus, a bacterial host such as E. coli may be treated with a solution of $CaCl_2$ (Cohen et al, Proc. Nat. Acad. Sci., 1973, 69, 2110) or with a solution comprising a mixture of RbCl, $MnCl_2$, potassium acetate and glycerol, and then with 3-[N-morpholino]-propane-sulphonic acid, RbCl and glycerol. Mammalian cells in culture may be transformed by calcium co-precipitation of the vector DNA onto the cells. The invention also extends to a host cell transformed with a replicable expression vector of the invention.

Culturing the transformed host cell under conditions permitting expression of the DNA polymer is carried out conventionally, as described in, for example, Maniatis et al and ''DNA Cloning'' cited above. Thus, preferably the cell is supplied with nutrient and cultured at a temperature below 45°C

The modified t-PA expression product is recovered by conventional methods according to the host cell. Thus, where the host cell is bacterial, such as E. coli it may be lysed physically, chemically or enzymatically and the protein product isolated from the resulting lysate. Where the host cell is mammalian, the product may generally be isolated from the nutrient medium. The DNA polymer may be assembled into vectors designed for isolation of stable transformed mammalian cell lines expressing the modified t-PA; e.g. bovine papillomavirus vectors (DNA cloning Vol.II D.M. Glover Ed. IRL Press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al., European Patent Application No. 0093619, 1983).

It will be appreciated that, depending upon the host cell, the modified t-PA prepared in accordance with the invention may be glycosylated to varying degrees. Furthermore, as observed by Pohl et.al., Biochemistry, 1984, 23, 3701-3707, varying degress of glycosylation may also be found in unmodified, naturally occurring t-PA. The modified t-PA of the invention is understood to include such glycosylated variations.

A particularly useful oligodeoxyribonucleotide for use in the process is of sequence (I):

    5'd(AGCCCTCCTATGATGC)3'                    (I)

The oligodeoxyribonucleotide of sequence (I) is novel and as such forms part of the invention.

The oligodeoxyribonucleotide of sequence (I) is complementary to the nucleotides 1013-1028 inclusive of t-PA cDNA except for one mismatch at the nucleotide at position 1019.  The codon at position 1018-1020 has thus been changed from AAA to ATA.  Consequently it is believed that the cDNA obtained following mutagenesis is substantially similar to the cDNA reported by Pennica et al 1983 with the following new sequence:-

              5´ -GCATCA T AGGAGGGCT- 3´
                    |        |          |
                  1013   position    1028
                          1019
                         changed

Expression of the cDNA is believed to give rise to t-PA in which the amino acid sequence is changed from

- 14 -

position        275      277          280
              -arg-ile-lys-gly-gly-leu-

                        to

position        275      277          280
              -arg-ile-ile-gly-gly-leu-

The modified t-PA of the invention is preferably administered as a pharmaceutical composition for the treatment of thrombotic disease.

Accordingly the present invention also provides a pharmaceutical composition comprising modified t-PA of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile modified t-PA in sterile isotonic aqueous buffer. Where necessary the composition may also include a solubilising agent to keep the modified t-PA in solution and a local anaesthetic such as lignocaine to ease pain at the site of injection. Generally, the modified t-PA will be supplied in unit dosage form for example as a dry powder or water free concentrate in a hermetically sealed container such as an ampoule or sachette indicating the quantity of protein in activity units. Where the modified t-PA includes a removable blocking group an indication of the time within which the free

- 15 -

protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration.

The quantity of material administered will depend upon the amount of fibrinolysis required and the speed with which it is required, the seriousness of the thromboembolic condition and position and size of the clot. The precise dose to be employed and mode of administration must <u>per force</u> in view of the nature of the complaint be decided according to the circumstances by the physician supervising treatment. However, in general, a patient being treated for a thrombus will generally receive a daily dose of from 0.01 to 3.0 mg/kg$^{-1}$ of body weight either by injection or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of modified t-PA of the invention.

The following Examples illustrate the invention.

Example 1

5'd(AGCCCTCCTATGATGC)3'

The above mentioned 16-mer(hexadecamer) was prepared by the solid phase phosphotriester method on a polydimethylacrylamide-kieselguhr support using monomer building blocks following the standard procedure described in 'Chemical and Enzymatic Synthesis of Gene Fragments - A Laboratory Manual' (ed. H.G. Gassen and A. Lang), Verlag Chemie, Weinheim (1982) Chapter 1, or M.J. Gait, H.W.D. Matthes, M. Singh, B.S. Sproat, and R.C. Titmas, Nucleic Acids Research, 1982, 10, 6243.

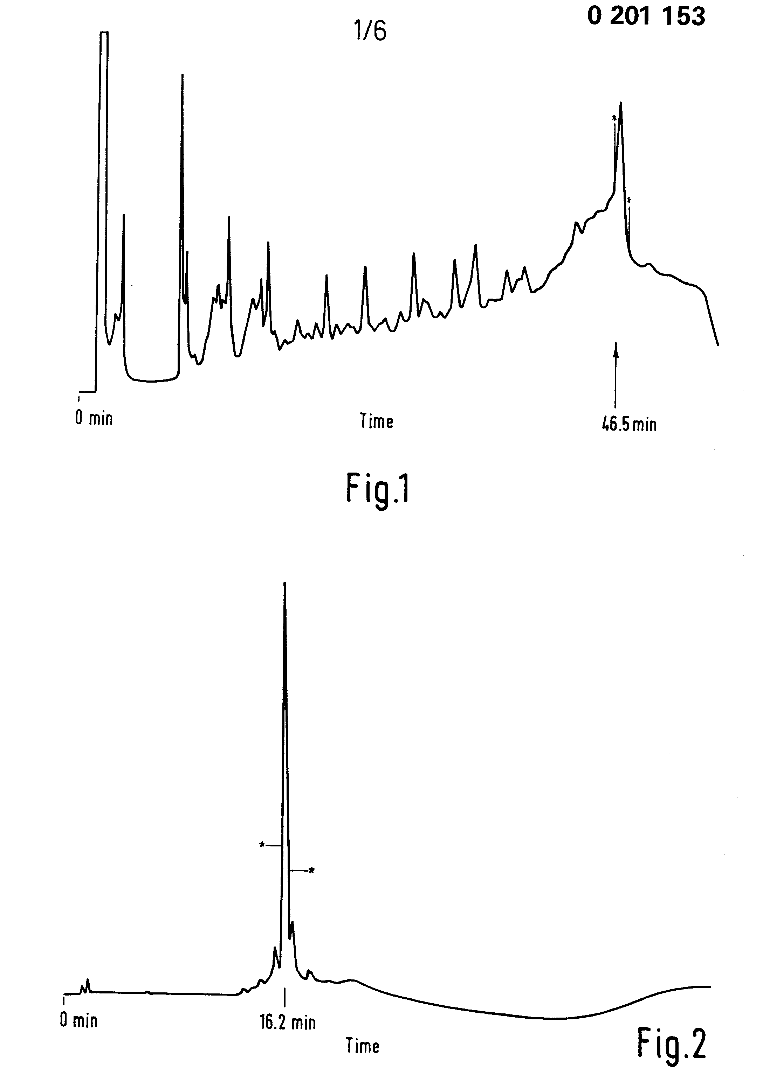

The product was purified by ion-exchange HPLC (Fig 1) followed by reversed phase HPLC (Fig 2) using standard methodology given in the above mentioned references.

Sequence verification was carried out by the Method of Maxam and Gilbert (see A.M. Maxam and W. Gilbert, 'Sequencing End-Labeled DNA with Base-Specific Chemical Cleavages' in Methods in Enzymology, ed. L. Grossman and K. Moldave, Academic Press Inc. (London) Ltd. (1980), Vol. 65, p.p. 499-560.

The length of the oligonucleotide was verified by a sizing experiment in which the electrophoretic mobility of the oligonucleotide was compared with that of oligo (dT) fragments contained in a commercially available oligo (dT) ladder.

Methods used for Examples 2 to 4

DNA cleavage

In general the cleavage of about 1µg of plasmid DNA or DNA fragments was effected using about 5 units of a restriction enzyme (or enzymes) in about 20µl of an appropriate buffer solution.

Generation of blunt ends:  If blunt ends were required they were produced by treating the DNA preparation with DNA Polymerase I, Klenow fragment (Molecular Cloning, A Laboratory Manual, Cold Spring Harbor Laboratory 1982).

5' end labelling of oligonucleotides:  Addition of radiolabelled phosphate groups to oligonucleotides was carried out as described (Maxam and Gilbert, Methods in Enzymology Vol.65 p499-560  Ed. Grossman and Moldane publisher Academic Press 1980).

Ligation of DNA Fragments:  Ligation reactions were carried out as described (Maniatis et al Molecular Cloning - A Laboratory Manual, Cold Spring Harbor Laboratory 1982).

Transformation of M13 DNA into bacterial cells was carried out using treatment with calcium chloride (Cohen et al 1973, P.N.A.S. 69, 2110).

Transformation of plasmid DNA into E. coli HB101 cells was as described by Hanahan (DNA Cloning Vol I Chapter 6, D.M. Glover ed. IRL Press, 1985) in Protocol 3 except that incubation with RFI was for 5 minutes.


Isolation of t-PA cDNA clones

RNA was isolated from the TRBM6 cell line (Browne et al 1985, Thrombosis and Haemostasis; 54; 422-424) using hot phenol/SDS extraction (M.R.D. Scott, 1982, Ph.D. Thesis, University of London) and messenger RNA purified using oligo dT cellulose chromatography. A TRBM6 cDNA library was established by synthesizing double-stranded cDNA using this messenger RNA essentially as described by M.R.D. Scott (Ph.D. Thesis 1982, University of London). This cDNA preparation was digested with BglII, size fractionated on a Biogel A150 column and ligated into a pAT153 vector which had been modified by introduction of a BglII linker at the NruI site; the resultant DNA clone was propagated in E. coli K12 DH1 cells. A t-PA-specific oligonucleotide probe (Browne et al, 1985; Gene 33, 279-284)was used to identify a t-PA cDNA clone in the cDNA library, this clone is known as pTR108, and carries a 2kb BglII fragment encoding the mature t-PA protein (Pennica et al, 1983, Nature, 301, 214-221).


A further cDNA clone λTR10, was isolated from a second TRBM6 cDNA library. The cDNA library was constructed using the λgt10 vector as described in 'DNA Cloning' Volume I, Chapters 2 and 3 (Ed. D.M. Glover (IRL Press, 1985)). The library was screened using appropriate t-PA specific oligonucleotide and plasmid probes using

- 19 -

methods described previously (Browne et al, 1985, Gene, 33, 279-284). Clone λTR10 isolated from this library carries additional DNA 5' to that in pTR108, corresponding to the prepro-coding region and part of the 5' untranslated region (Pennica et al, 1983, Nature, 301, 214-221).

Growth of M13 single strand DNA: A single M13 phage plaque was picked into a 1:100 dilution in 2YT (1.6% Bactotryptone, 1% Yeast extract, 1% NaCl) of a fresh overnight culture of E. coli strain BMH 71-18 (Gronenborn et al, 1976, Mol. Gen. Genet. 148, 243-250). The culture was grown at 37°C with shaking for 5-6 hours. The bacteria were pelleted and the supernatant retained. To this was added 200µl of 2.5M NaCl, 20% PEG6000 and the mixture was incubated at room temperature for 15 minutes. The mixture was centrifuged for 5 minutes in an Eppendorf microfuge and the resulting phage pellet was resuspended in 100µl of 10mM Tris pH 7.9, 0.1mM EDTA. After phenol extraction the phage DNA was precipitated with ethanol. The DNA pellet was washed with 70% ethanol, air dried and resuspended in 30µl of 10mM Tris pH 7.9, 0.1mM EDTA.

Growth of double stranded M13 DNA: A single M13 phage plaque was picked into 1ml of 2YT and grown with shaking at 37°C for 6 hours. The bacteria were pelleted and the supernatant containing M13 phage retained. Meanwhile a one litre 1:100 dilution of an overnight culture of E. coli strain BMH 71-18 was grown at 37°C with shaking for 2 hours. 500µl of the M13 phage supernatant was added and the culture was shaken at 37°C for a further 4 hours. Preparation of double

- 20 -

stranded DNA was carried out as described by Maniatis _et al_ (Molecular Cloning, A Laboratory Manual, Cold Spring Harbor Laboratory, 1982).

_Site directed mutagenesis_: The site-directed mutagenesis reactions were carried out essentially as described by Carter _et al_ (Oligonucleotide site-directed mutagenesis in M13, An Experimental Manual, Anglian Biotechnology Ltd., 1985).

The following priming mixture was set up in a total volume of 10µl: 1µg template DNA (mTR30 single stranded form, see Example 2); 3µg kinased mutagenic oligonucleotide primer (as described above); 1µl 10 x TM Buffer (100mM Tris pH 8.0, 100mM $MgCl_2$); 5.5µl $H_2O$. The mixture was sealed into a glass capillary, boiled for three minutes and allowed to cool to room temperature. To the priming mixture were then added:- 2µl 10 x TM buffer; 1µl of a mixture of 4mM each of dATP, dGTP, dCTP, dTTP; 1µl rATP; 1µl 100mM DTT; 12µl $H_2O$; 1 unit DNA Polymerase I (Klenow fragment); 7 units T4 DNA ligase giving a total final volume of 30µl. The mixture was incubated for 4h at 14°C.

The DNA was transformed in 3µl aliquots into E. coli strain BMH 71-18 mut L (Kramer _et al_, 1984, Cell **38**, 879-887). The transformed bacteria were plated onto a lawn formed from E. coli strain BMH 71-18. Some of the plaques resulting from the transformations were picked and plated to form duplicate gridded arrays of bacterial colonies. These were lifted onto nitrocellulose and lysed as described (Grunstein and Hogness, 1975, P.N.A.S. **72**, 3961).

- 21 -

Screening conditions: The nitrocellulose filters were prehybridised for 3h at 30°C in 6 x SSC (1 x SSC is 150mM NaCl, 15mM Na$_3$C$_6$H$_5$O$_7$2H$_2$O) 0.1% SDS, 10 x Denhardt's (Denhardt's is 0.02% Polyvinyl-pyrrolidone, 0.02% Ficoll, 0.02% BSA), 50μg sonicated salmon sperm DNA. The filters were then mixed with radioactively labelled mutagenic primer under the same buffer conditions and were hybridised overnight at 30°C.

The filters were washed at a series of different temperatures in 6 x SSC plus 0.1% SDS: 15 minutes at 30°C; 3 minutes at 44°C; 3 minutes at 49°C; 3 minutes at 51°C; 3 minutes at 54°C. After each wash the filters were exposed, wet (with an intensifying screen) to Fuji RX-100 X-ray film.

Sequencing: DNA sequencing was carried out by the dideoxy termination method (Sanger, Nicklen and Coulson, 1977, P.N.A.S. 74, 5463).

Plasmid preparation: Large scale preparation of plasmid DNA and plasmid mini-preparations were carried out as described in Maniatis et al (Molecular Cloning - A Laboratory Manual, Cold Spring Harbor Laboratory, 1982).

Isolation of DNA fragments from low-melting-point (LMP) agarose gels

DNA fragments were isolated from LMP agarose gels as described by Maniatis et al (Molecular Cloning, A Laboratory Manual, 1982, Cold Spring Harbor).

- 22 -

Ligation of Synthetic Linkers to DNA

Synthetic linkers encoding restriction enzyme sites, were kinased and ligated to blunt-ended DNA as described by Maniatis et al (Molecular Cloning, A Laboratory Manual, 1982, Cold Spring Harbor).

Dephosphorylation of DNA

Vector DNA was dephosphorylated, where appropriate, by treatment with calf intestinal alkaline phosphatase as described by Maniatis et al (Molecular Cloning, A Laboratory Manual, 1982, Cold Spring Harbor).

Fibrin/agar overlay for detection of t-PA expression

Cell preparation: cells were trypsinised and plated out at $10^6$ cells per 60mm dish and left overnight in growth medium (10% Serum, 1% stock solution of penicillin/ streptomycin; Flow Laboratories, 1% Glutamine, 1% stock solution of non-essential amino acids; Flow Laboratories, in Eagles MEM) at 37°C in a humidified incubator in an atmosphere of 5% $CO_2$/95% air.

Transfection procedure: The transfection used (calcium coprecipitation) is described in 'DNA Cloning' Ed. D.M. Glover (Chap. 15, C. Gorman). Glycerol shock and 10mM butyrate treatment were used. Following transfection the cells were rested overnight in growth medium.

Overlay: Agarose (Indubiose $A_{37}$), 2.4g in 95ml Eagles MEM (Gibco) heated to melting point in a bunsen flame, was then placed in a water bath maintained at 48°C.

5.6 ml of fibrinogen (20mg/ml) were diluted 1:1 with
5.6 ml of Eagles MEM (containing an extra 7 mg/ml of
NaCl) and retained on ice. 3.3 ml of Eagles MEM (no
additions) were aliquoted into a bijou containing 86μl
of bovine thrombin at 50 NIH Units/ml (retained on
ice). The cells were washed 3 times with Eagles MEM to
remove serum. The thrombin and fibrinogen were warmed
to 37°C in a water bath. 9.5 ml agarose were aliquoted
into a pre-warmed universal. The thrombin was added to
the agar, followed by the fibrinogen. The gel was
poured over the cell layer and incubated at 37°C until
lysis zones appeared.


Preparation of modified t-PA from bacterial extracts


Modified t-PA was extracted from cultures of E. coli
JM101 (pTRB8) using a procedure based on that described
by Heynecker et al, (1983) European Patent Application
No. 0092182 for the extraction of active urokinase from
E. coli cells. 10ml prewarmed L-broth (5g NaCl, 5g
Yeast Extract, 10g Tryptone/litre of water) containing
50μg/ml ampicillin, were inoculated with 100μl of a
standing overnight culture of E. coli JM101 (pTRB8) and
incubated, with aeration, at 37°C until the $A_{550} \simeq 0.5$.
Expression of modified t-PA was then induced with
0.1mM IPTG (isopropyl β-D-thiogalactopyranoside) and
$ZnSO_4$ was added to a final concentration of 0.5mM to
minimise bacterial protease activity (Gross et al,
Biochim. Biophys. Acta. 825, 207-213, 1985). Growth
was continued for a further 2h (final $A_{550} \simeq 1.0$-1.2)
and the cells placed in an ice bath. The cells were
harvested, washed once with 1 volume ice-cold 10mM
phosphate buffer pH 7.0 and the pellet resuspended in
1ml lysis buffer (50mM Tris pH 8.0, 50mM EDTA, 2mg/ml

— 24 —

lysozyme).  After 30 min at 0°C, 3 ml 6M guanidine HCl, 50mM Tris pH 8.0 were added and mixed well.  The cell debris was removed by  centrifugation at 12,000g for 30 mins at 4°C and the supernatant decanted and incubated at 24°C for 30 mins.  The solution was subsequently diluted to 1M guanidine HCl, 50mM Tris pH 9.0 and made 2mM reduced glutathione (GSH), 0.2mM oxidized glutathione (GSSG).  This redox mixture was incubated at 14°C for 16h and then dialysed against 50mM Tris pH 9.5, 250mM NaCl, 0.25mM EDTA, 0.01% Tween for 8h (one change of buffer).  10µl of the dialysed extract were placed onto a fibrin plate (Robinson, J. Thrombos. Res. 1984; 33, 155-62) together with standards.

Example 2

Change in t-PA DNA sequence

DNA coding for a novel, modified t-PA protein has been produced using the above mentioned oligonucleotide (Example 1) with sequence:-

5' d(A G C C C T C C T A T G A T G C)3'

This oligonucleotide is complementary to nucleotides 1013-1028 inclusive of t-PA cDNA (Pennica et al, Nature, 1983, 301, 214-221) except for one mismatch at position 1019.  The codon at position 1018-1020 has thus been changed from AAA to ATA.  The resulting DNA sequence has been expressed in both prokaryotic and eukaryotic cell systems to give a novel fibrinolytically active protein.

All manipulation of the DNA referred to below was carried out as described in the Methods Section.

A cDNA clone coding for the protein t-PA was isolated from mRNA prepared from the TRBM6 cell line described in Thrombosis and Haemostasis, 1985, <u>54</u>, 422-424. The 2kb BglII fragment encoding the mature t-PA cDNA sequence was isolated, blunt-ended and subcloned into M13 mp10 (Amersham International PLC Prod. No. N.4536) at the SalI site which had also been blunt-ended. Clones were isolated with the insert in both orientations. The two types of clone will henceforth be known as mTR10 and mTR20. One of these, mTR20, placed the t-PA cDNA insert 'in frame' and in the correct orientation, to allow expression of a t-PA fusion protein from the lacZ promoter in the M13 mp10 genome (Slocombe <u>et al</u>, 1982, P.N.A.S. <u>79</u>, 5455-5459).

This construct did not give good yields of either single or double stranded DNA.

The t-PA cDNA was put out of reading-frame by cutting double stranded DNA from the clone mTR10 with the enzymes BamHI and HindIII. The whole DNA mixture comprising the cleaved vector DNA and the t-PA insert DNA was blunt-ended and religated. The resulting construct was shown by DNA sequencing to have the following structure at the point of insertion indicating that the t-PA cDNA is out of reading-frame with respect to the M13 lacZ promoter.

```
        mp10 derived sequence            t-PA sequence

5' GGG GAT CAG CTT GGG CTG CAG GTC GA    |/GA TCT 3'

           ───────────►                  ↓

           transcription                 BglII
```

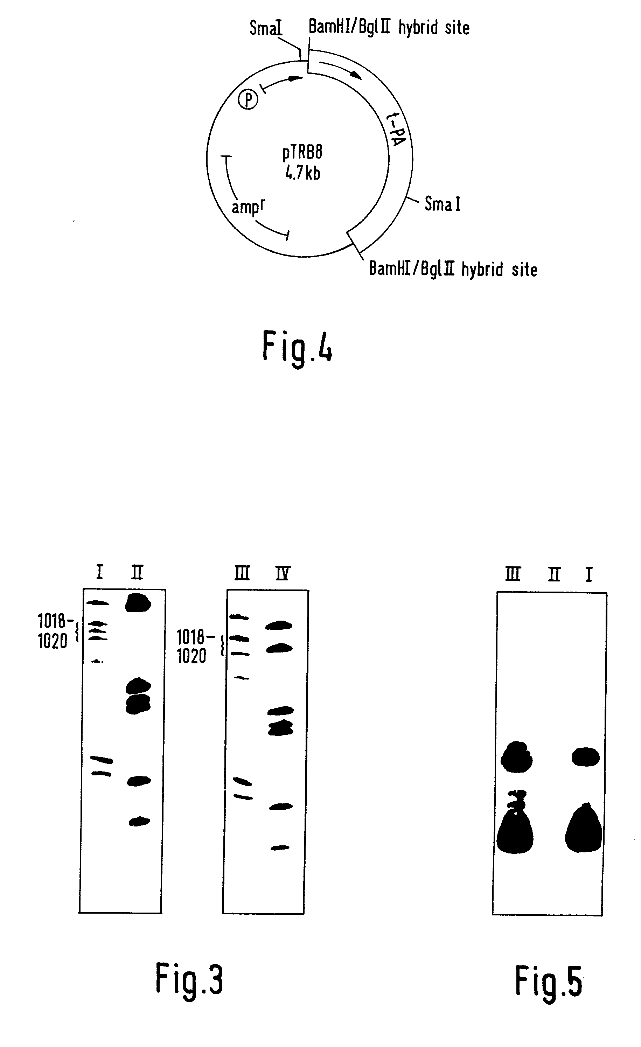

This clone will be known as mTR30. Site directed mutagenesis was carried out on the clone mTR30 as described in Methods. After screening, five duplicate positive signals were selected. DNA sequencing was carried out on single stranded DNA from the five isolates and all clearly showed a changed DNA sequence. An example of this changed DNA sequence from the mutant clone mTR40 is shown in Fig. 3.

The tracks shown in Fig. 3, I, III represent terminations at all positions where the base A is present in the DNA sequence. The tracks shown in Fig. 3, II, IV represent terminations at all positions where the base T is present in the DNA sequence. Fig. 3, tracks I, II are derived from normal t-PA DNA. Fig. 3, III, IV are derived from the novel t-PA DNA. In each case the triplet 1018-1020 is indicated. The change from the sequence AAA to ATA was therefore confirmed.

Example 3

Bacterial Expression of modified t-PA DNA

The 2kb BglII fragment carrying the modified t-PA sequence was excised from mTR40 by restriction with BglII. This was subsequently ligated with BamHI cut pUC8. Competent E. coli JM101 cells were transformed with 0.1μg ligated DNA and after a 60 min incubation in L-broth (LB) to allow expression of plasmid encoded β-lactamase, transformants were selected on L-agar (LB +1.5% Bacto agar) incorporating ampicillin at 50μg/ml and X-gal (5-bromo, 4-chloro, 3-indolyl-β-D-galactopyranoside) a chromogenic β-galactosidase substrate, at 20μg/ml. Several white colonies were purified and screened for the presence of the required recombinant plasmid (4.7kb). This was carried out by preparing plasmid mini-preps based on the procedure of Birnboim and Doly, 1979, Nucleic Acids Research 7(6) p1513-1523). Those isolates demonstrated to be carrying a 4.7kb plasmid were restricted with SmaI to determine the orientation of the modified sequence with respect to the lacZ promoter of the pUC8 vector. One recombinant shown to carry the BglII fragment in the correct orientation for expression of the modified t-PA sequence was chosen for further study. This expression plasmid, pTRB8, has the structure shown in Fig.4.

Modified t-PA was expressed from the fused lacZ/t-PA coding DNA in E. coli JM101 as described in the methods section. Analysis of the dialysed extract reveals a major band of fibrinolytic activity apparently identical in size to unmodified material from E. coli using the described protocol (Fig.5). Several bands of lower apparent molecular weight are visible on zymography of modified and unmodified material, these are believed to be either degradation products or the result of internal translation initiation events on the mRNA template. No fibrinolytic activity was detected in extracts from E. coli JM101 (pTRB9) (a plasmid construction carrying the modified t-PA sequence in the opposite orientation with respect to the lacZ promoter of the vector).

Example 4

Transient Expression of modified t-PA DNA in Eukaryotic Cells

A.  Vector Construction

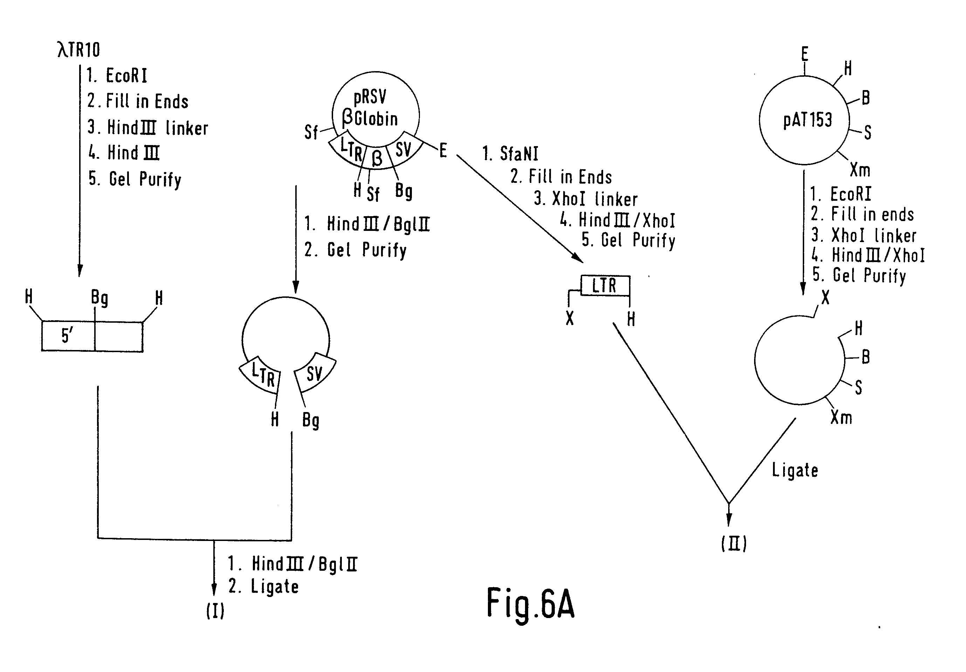

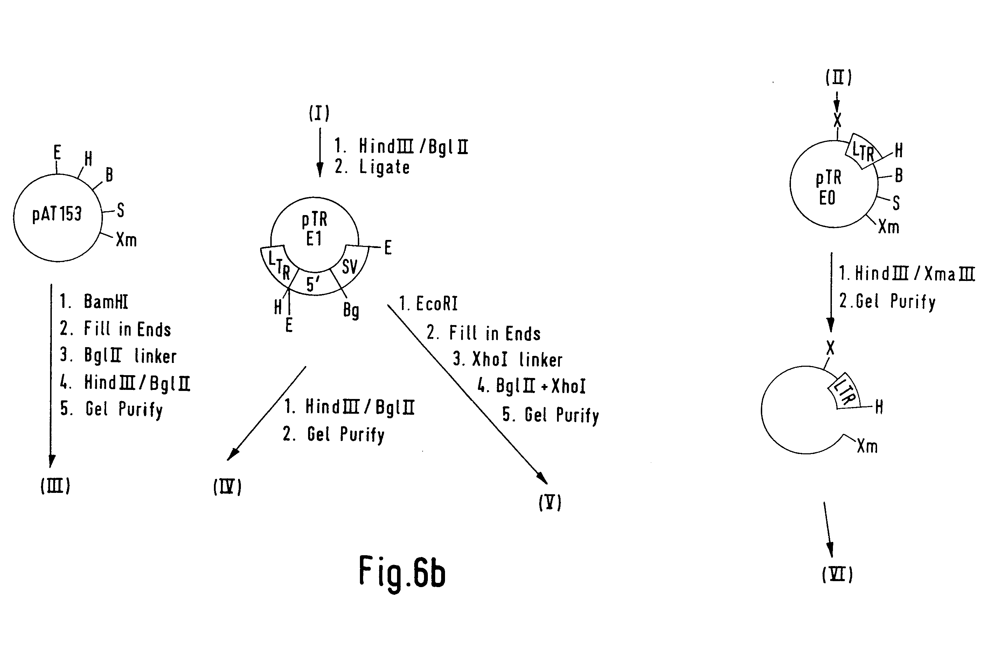

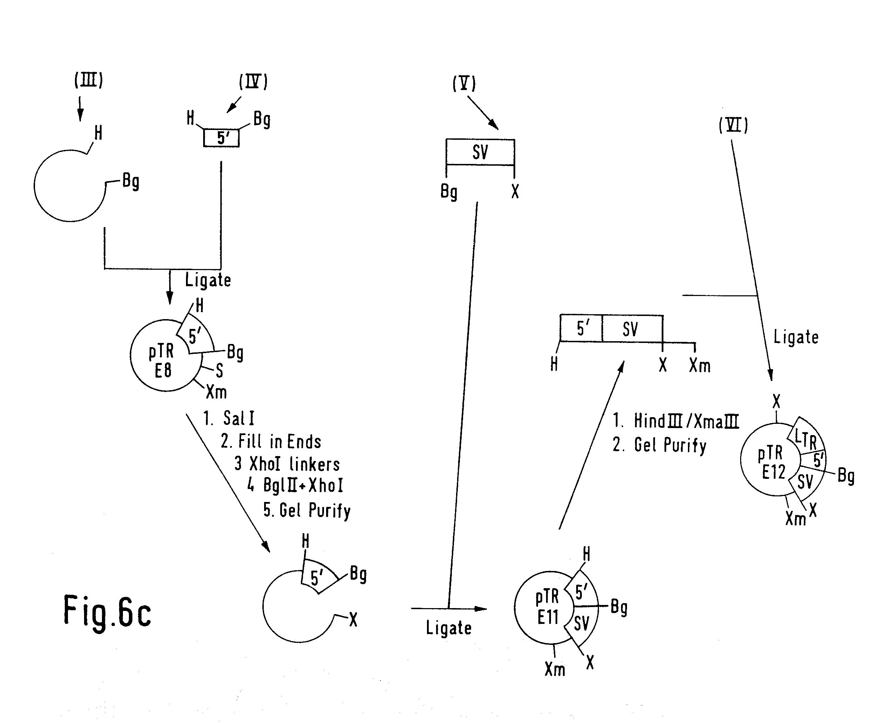

The vector pRSV-β-globin (Gorman et al, Science, 221, 551-553, 1983) was modified to allow the insertion and expression of t-PA cDNA (see Fig.6).  The β-globin sequences were removed by digesting 10µg of pRSV-β-globin DNA with 20 units each of HindIII and BglII for 2h and isolating the large DNA fragment from a 1% low melting point agarose gel as described in the methods section.  The β-globin sequences were replaced with a DNA fragment encoding the 5' region of the cDNA from λTR10 which is a λgt10 cDNA clone ('DNA Cloning, Vol.I, chapters 2 and 3, Ed. D.M. Glover, IRL Press, 1985) constructed using TRBM6 cell messenger RNA (Thrombosis and Haemostasis, 1985, 54, 422-424).  The cDNA insert in the λTR10 encodes the 5' untranslated region, prepro sequences and mature protein sequences up to the EcoRI site equivalent to base 801 (Pennica et al, Nature, 1983, 301, 214-221).  The cDNA insert was cleaved from the vector by digestion of 5µg of DNA with 24 units of EcoRI for 1.5h.  The DNA was then extracted with phenol/ chloroform, ethanol precipitated, resuspended, blunt-ended and ligated to kinased HindIII linkers as described in the Methods section.  Ligation of linkers was at ambient temperature for 7 hours then overnight at 4°C.  The DNA was then digested with 20 units of HindIII for 2 hours and a  800bp fragment corresponding to the HindIII-linkered t-PA cDNA was isolated after electrophoresis in a 1% low melting point agarose gel. This  800bp fragment was mixed with the large DNA fragment derived from pRSV-β-globin described above and ethanol precipitated. The DNA was resuspended and digested with 10 units each of HindIII and BglII for 1.7h, phenol/chloroform extracted, ethanol precipitated, resuspended and ligated as described in the methods section for 5h at ambient temperature and overnight at 4°C. 2μl of DNA was used to transform E. coli HB101 and colonies carrying plasmids containing the 5' end of the t-PA cDNA up to the BglII site equivalent to that numbered 187 in Pennica et al (Nature, 1983, 301, 214-221), were identified by HindIII/BglII digestion of plasmid mini-preps and the plasmid named pTRE1.

The sequences encoding the Rous sarcoma virus long terminal repeat (RSV-LTR), 5' region of t-PA cDNA, and Simian virus 40 (SV40) 3' sequences were then reconstructed in pAT153 in such a way that they formed a single XhoI fragment. Firstly, the RSV LTR was removed from pRSV-β-globin by digestion of 6μg DNA with 4U of SfaNI for 2.5h. The DNA was then phenol/chloroform extracted, ethanol precipitated, resuspended and blunt-ended. XhoI linkers were kinased and ligated to the DNA at ambient temperature for 8h and overnight at 4°C, then incubated at 70°C for 15 minutes to inactivate the ligase and ethanol precipitated. The linkered DNA was then resuspended and digested with 15 units each of HindIII and XhoI for 6h and a ~500 bp fragment corresponding to the RSV-LTR isolated after electrophoresis in a 1% LMP agarose gel. This DNA fragment was cloned into pAT153 prepared as follows: 6μg of pAT153 was digested with 30U of EcoRI for 2h, then phenol/chloroform extracted and ethanol precipitated. The DNA was resuspended, blunt-ended, ligated to XhoI linkers, resuspended and digested with HindIII and XhoI as above. A 3.4kb fragment was isolated after electrophoresis in a 1% LMP agarose gel and ethanol precipitated with the ∿ 500bp fragment above. The DNA was ligated to give pTRE0.

The ∿0.2kb fragment encoding the 5' part of the t-PA cDNA was isolated from pTRE1 by digesting 10μg of DNA with 30 units each of HindIII and BglII for 2 hours and the fragment isolated after electrophoresis in a 1% LMP agarose gel. This fragment was cloned into pAT153 prepared as follows: 5μg of pAT153 was digested to completion with BamHI. Following phenol/chloroform extraction and ethanol precipitation, the DNA was resuspended and blunt-ended then ligated to kinased BglII linkers. The DNA was phenol/chloroform extracted, ethanol precipitated, resuspended and digested with 30 units each of HindIII and BglII for 3h. The large DNA band was isolated after electrophoresis in a 1% LMP agarose gel, ethanol precipitated with the ∿0.2kb fragment above and ligated together to give pTRE8.

A DNA fragment encoding the SV40 sequences from pTRE1 was isolated as follows: 5μg of pTRE1 was digested with 60 units of EcoRI for 2h, blunt-ended and ligated to XhoI linkers as described previously. After phenol/chloroform extraction and ethanol precipitation, the DNA was resuspended and digested sequentially with BglII and XhoI. A ∿1.6kb band encoding the SV40 sequences was isolated after electrophoresis in a 1% LMP agarose gel and cloned into pTRE8 prepared as follows: 5μg of pTRE8 was digested with 15 units of SalI for 3½h, blunt-ended and ligated to XhoI linkers as described previously. Following phenol/chloroform extraction and ethanol precipitation, the DNA was

- 31 -

resuspended and digested sequentially with 30 units of BglII for 1h and 10 units XhoI for 2h. The large DNA band was isolated after electrophoresis in a 1% LMP agarose gel, ethanol precipitated with the 1.6kb fragment above and ligated to give pTRE11.

A ~2kb DNA fragment encoding the 5' t-PA cDNA sequences, SV40 sequences and part of pAT153 were cleaved from pTRE11 by digestion of 5µg DNA with 30 units HindIII and 6 units XmaIII for 6h. After electrophoresis in a 1% LMP agarose gel a ~2kb band was isolated and cloned into pTRE0 prepared as follows: 4.8µg pTRE0 was digested with HindIII and XmaIII as above and the large DNA fragment isolated after electrophoresis in a 1% LMP agarose gel. This fragment was ethanol precipitated with the 2kb fragment above and ligated to give pTRE12.

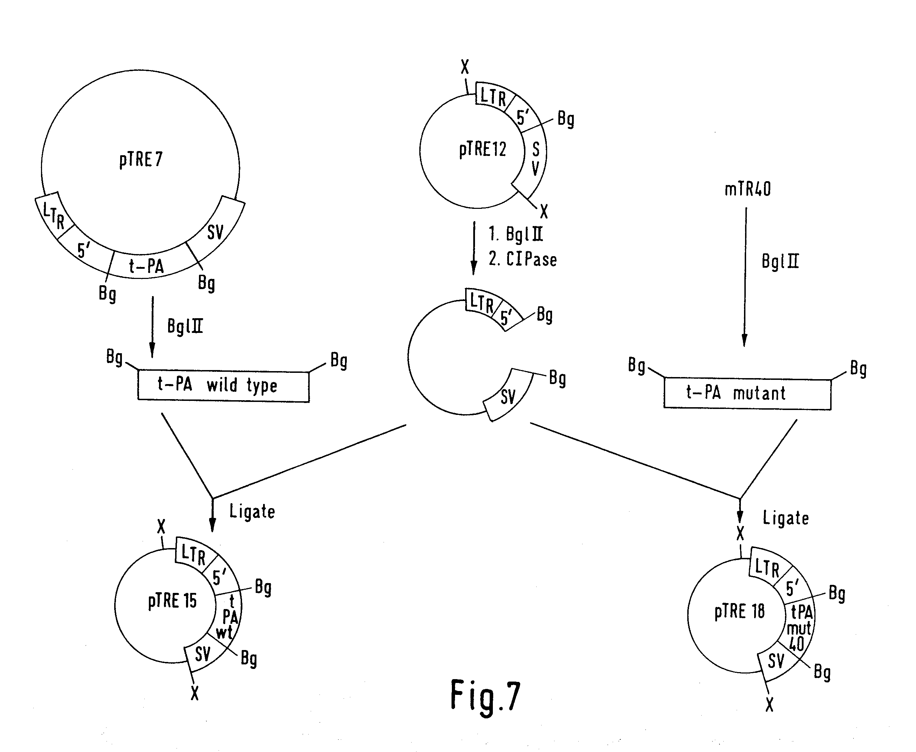

t-PA cDNA BglII fragments were cloned into the unique BglII site of pTRE12 as shown in Figure 7. pTRE7 contains a 2kb BglII fragment, encoding the mature t-PA protein and part of the 3' untranslated region, derived from a cDNA library produced from TRBM6 cells, (Thrombosis and Haemostasis, 1985, 54, 422-424), cloned into the unique BglII site of pTRE1. 5µg of pTRE7 was digested to completion with BglII and the ~2kb fragment isolated after electrophoresis in a 1% LMP agarose gel. This was ethanol precipitated and ligated with pTRE12, previously digested with BglII and phosphatased as described in the methods section, to give pTRE15 which expresses wild-type t-PA. mTR40 was digested with BglII and a 2kb fragment isolated and cloned into pTRE12 as described above to give pTRE18 which expresses mutated t-PA.

- 32 -

B.   Fibrin/agar overlay

Expression of modified and unmodified t-PA from transfected mouse C127 cells (24 hours post-transfection) was detected using the fibrin/agarose overlay technique as described in the Methods section.  Lytic zones were obtained from pTRE15 (unmodified t-PA) and pTRE18 (modified t-PA) transfected cells, but not from cells transfected with the empty parent vector pTRE12 (t-PA$^-$).

In the figures:

Figure 1:   Example 1a Analytical ion-exchange HPLC

Conditions:  Partisil 10-SAX column eluted at 2ml/min with 0.001 M and 0.3 M KH$_2$PO$_4$, pH 6.3, in 6:4 formamide-water (solvents A and B respectively), using a gradient of 0-100% B over 50 min at room temperature.  Detection was at 270 nm.

Note:  In preparative runs, the peak eluting at 46.5 min was collected between the points indicated by asterisks.

Figure 2:   Reversed phase HPLC on purified 16-mer of Example 1a

Conditions:  μ-Bondapak C-18 column eluted at 2 ml/min with 0.1 M ammonium acetate (solvent A) and 0.1 M ammonium acetate-acetonitrile (2:8 v/v, solvent B), using a gradient of A + 0-15% B over 30 min at room temperature.  Detection was at 270 nm.

Note: In preparative runs, the material responsible for the major peak was collected between the points indicated by asterisks.

Figure 3: DNA sequencing tracks

I, II: normal t-PA DNA A, T tracks respectively
III, IV: t-PA DNA of Example 1b A, T tracks respectively

Figure 4: Structure of bacterial expression plasmid pTRB8

(P)    - lac Z promoter (-> shows direction of transcription from this promoter)

$amp^r$ - β- lactamase gene

___    - pUC8 derived sequences

☐     - t-PA derived sequences (-> denotes orientation ie 5' -> 3')

The orientation of the t-PA encoding insert was determined by restriction with SmaI.

The expression plasmid carrying an unmodified t-PA insert, pTRB5, was constructed in a similar fashion.

Figure 5: Zymography of bacterial extracts

Activity was present in track I (modified t-PA DNA) and track III (unmodified t-PA DNA) but not in track II (modified t-PA DNA in reverse orientation with respect to promoter).

- 34 -

Figure 6:  Construction of vector (pTRE12) for
insertion and expression of t-PA sequences
Abbreviations:


λTR10        -       λgt10 clone containing the
5' part of                  t-PA cDNA


LTR          -       Rous sarcoma virus long terminal repeat


β            -       Rabbit β globin cDNA


SV           -       Simian virus 40 sequences including
                     small t antigen intron and early region
                     polyadenylation site


5'           -       5' part of t-PA cDNA (approximately
                     0.2kb long) with 3´ end equivalent to
                     the BglII site at position 187 in
                     Pennica et al Nature 301, 214-221, 1983


B            -       recognition site for BamHI

Bg           -       recognition site for BglII

E            -       recognition site for EcoRI

H            -       recognition site for HindIII

S            -       recognition site for SalI

Sf           -       recognition site for SfaNI

X            -       recognition site for XhoI

Xm           -       recognition site for XmaIII

**0 201 153**

Figure 7: <u>Construction of vectors expressing wild-type or mutant t-PA (pTRE15 and pTRE18 respectively)</u>

Abbreviations are as in Figure 6.  t-PA wild type is cDNA encoding normal t-PA, t-PA mutant is cDNA encoding altered t-PA.

CLAIMS

1.   A modified tissue-type plasminogen activator comprising an amino acid without a charged side chain in place of lysine at position 277.

2.   A modified tissue-type plasminogen activator according to claim 1, in which the amino acid without a charged side chain is isoleucine.

3.   A modified tissue-type plasminogen activator prepared according to Example 3 or 4.

4.   A derivative of the modified tissue-type plasminogen activator according to claim 1.

5.   A DNA polymer comprising a nucleotide sequence that encodes a modified tissue-type plasminogen activator according to any of claims 1 to 3.

6.   A replicable expression vector capable, in a host cell, of expressing a DNA polymer according to claim 5.

7.   A host cell transformed with the vector according to claim 6.

8.   An oligodeoxyribonucleotide which is complementary or corresponds to tissue-type plasminogen activator cDNA in the region on either side of the codon formed by the nucleotides 1018-1020 but which mismatches in the region 1018-1020 such that the triplet of the oligodeoxyribonucleotide in the mismatch region is complementary or corresponds to a codon which encodes an amino acid without a charged side chain.

9. An oligodeoxyribonucleotide according to claim 8, wherein said triplet in the mismatch region is complementary or corresponds to a codon which encodes isoleucine.

10. The oligodeoxyribonucleotide having the sequence (I):

5'd(AGCCCTCCTATGATGC)(I)

11. A pharmaceutical composition comprising modified tissue-type plasminogen activator according to any of claims 1 to 4 in combination with a pharmaceutically acceptable carrier.

12. A modified tissue-type plasminogen activator according to any of claims 1 to 4 for use as an active therapeutic substance.

13. A modified tissue-type plasminogen activator according to any of claims 1 to 4 for use in the treatment of thrombotic disease.

14. A process for preparing modified tissue-type plasminogen activator according to claim 1, which process comprises expressing DNA encoding said modified tissue-type plasminogen activator in a recombinant host cell and recovering the modified tissue-type plasminogen activator product.

15. A process for preparing a DNA polymer according to claim 5, by the condensation of appropriate mono-, di or oligomeric nucleotide units.

16. A process according to claim 15, which process comprises effecting a site-directed mutagenesis upon the cDNA which codes for tissue-type plasminogen activator.

0 min          Time          46.5 min

Fig.1

0 min          16.2 min          Time          Fig.2

SmaI   BamHI/BglII hybrid site

P

t-PA

pTRB8
4.7kb

ampr

SmaI

BamHI/BglII hybrid site

## Fig.4

I   II        III   IV

1018-
1020

1018-
1020

III   II   I

## Fig.3

## Fig.5

Fig.6A

Fig.6b

Fig.6c

Fig.7